(19) Europäisches Patentamt / European Patent Office / Office européen des brevets

(11) **EP 4 113 150 B1**

(12) **EUROPÄISCHE PATENTSCHRIFT**

(45) Veröffentlichungstag und Bekanntmachung des Hinweises auf die Patenterteilung:
**04.06.2025   Patentblatt 2025/23**

(21) Anmeldenummer: **21182276.2**

(22) Anmeldetag: **29.06.2021**

(51) Internationale Patentklassifikation (IPC):
*G01R 33/565* (2006.01)

(52) Gemeinsame Patentklassifikation (CPC):
**G01R 33/56554; G01R 33/56527**

(54) **KORREKTUR DER CHEMISCHEN VERSCHIEBUNGS- ARTEFAKTE AUS BIPOLAREN DIXON-MR AUFNAHMEDATEN UNTER VERWENDUNG EINES NEURONALEN FALTUNGSNETZWERKS (CNN)**

CORRECTION OF CHEMICAL SHIFT ARTIFACTS FROM BIPOLAR DIXON-MR IMAGING DATA USING A CONVOLUTIONAL NEURAL NETWORK (CNN)

CORRECTION DES ARTEFACTS CHIMIQUES DE DÉCALAGE À PARTIR DES DONNÉES D'ENREGISTREMENT BIPOLAIRE DIXON-RM À L'AIDE D'UN RÉSEAU NEURONAL À CONVOLUTION (CNN)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**04.01.2023   Patentblatt 2023/01**

(73) Patentinhaber: **Siemens Healthineers AG**
**91301 Forchheim (DE)**

(72) Erfinder: **Zeller, Mario**
**91054 Erlangen (DE)**

(74) Vertreter: **Siemens Healthineers**
**Patent Attorneys**
**Postfach 22 16 34**
**80506 München (DE)**

(56) Entgegenhaltungen:
**EP-A1- 3 796 023**

• **INTERNATIONAL SOCIETY FOR MAGNETIC RESONANCE IN MEDICINE, ISMRM, 2030 ADDISON STREET, 7TH FLOOR, BERKELEY, CA 94704 USA, no. 5608, 1 June 2018 (2018-06-01), XP040704816**

• **INTERNATIONAL SOCIETY FOR MAGNETIC RESONANCE IN MEDICINE, ISMRM, 2030 ADDISON STREET, 7TH FLOOR, BERKELEY, CA 94704 USA, no. 4014, 26 April 2019 (2019-04-26), XP040711398**

## Beschreibung

Die vorliegende Erfindung betrifft ein Verfahren zur Korrektur chemischer Verschiebungs-Artefakte, sogenannter "Chemical Shift Artefacts" (CSA), die im Magnetresonanz-DIXON-Verfahren beim Verwenden von bipolaren Auslesegradienten (bipolares DIXON-MR) zur Erfassung der gleich- und gegenphasigen Echos auftreten.

Die vorliegende Erfindung liegt auf dem Gebiet der Kernspintomographie (Synonym: Magnetresonanztomographie, MRT), wie sie in der Medizin zur Untersuchung von Patienten Anwendung findet. Die Kernspintomographie basiert auf der Anwendung von Spinecho- und Gradientenechosequenzen, die bei einer gegebenen Messzeit eine exzellente Bildqualität ermöglichen.

Die Erfindung findet insbesondere Anwendung bei Verfahren, die einen binäre Auslesegradienten verwenden, wie z.B. die Magnetresonanz (MR)-Messung nach dem von Dixon 1984 veröffentlichten MR-Bildgebungsverfahren mit dem sich Wasser- und Fettgewebe getrennt darstellen lassen.

In seiner ursprünglichen Implementierung nahm Dixon ein Bild mit Wasser- und Fettsignalen in Phase und ein weiteres Bild mit Wasser- und Fettsignalen um 180° phasenverschoben auf. Dixon zeigte, dass eine einfache Summierung und Subtraktion der beiden Bilder ein reines Wasserbild bzw. ein reines Fettbild ergeben kann. Die DIXON Methode bzw. Technik kodiert im Wesentlichen die chemische Verschiebungsdifferenz in die Signalphase mit einer modifizierten Datenerfassung und erreicht dann die Wasser/Fett-Trennung durch Nachbearbeitung.

Die Magnetresonanz-Methode nach DIXON ist allgemein bekannt und wird mittlerweile auch im klinischen Alltag verwendet. Details zur Durchführung der Methode nach DIXON sind bekannt und werden z.B. in dem Übersichtsartikel von 2008 von Jingfei Ma beschrieben (Jingfei Ma, "Dixon Techniques for Water and Fat Imaging", Journal of Magnetic Resonance Imaging, 28, 543-558 (2008)).

Es ist bekannt und auch von Jingfei Ma (2008) beschrieben, dass das MR-Verfahren nach DIXON zu chemischen Verschiebungs-Artefakten führt, die durch den Einfluss der chemischen Verschiebung an den Grenzschichten zwischen Fett und Wasser entstehen. Diese Artefakte zu verhindern oder zu minimieren, ist Gegenstand von Forschung wie z.B. in DE 10 2008 057 294 A und EP 3 796 023 A1 beschrieben.

Die EP 3 796 023 A1 beschreibt z.B. ein Verfahren, das eine Verringerung der Bildqualität, und dadurch das Auftreten störender Artefakte vermeidet, in dem die Gradientenpolarität bei der DIXON Aufnahme mit bipolaren Auslesegradienten zwischen einzelnen Wiederholungen umgeschaltet wird. Diese Methode ist jedoch nur anwendbar, wenn mehrere Mittelwerte erfasst werden.

Ein MRT-Scan kann bis zu 90 Minuten dauern, was ihn für manche Anwendungen bzw. Patienten unpraktikabel macht, da man für diesen langen Zeitraum still liegen muss. Typischerweise können z.B. Babys oder Menschen, die an der Parkinson-Krankheit oder Klaustrophobie leiden, nicht so lange im Scanner bleiben, ohne sich einer Vollnarkose zu unterziehen. Es ist somit grundsätzlich ein Ziel, die Aufnahmezeit bei guter Qualität so gering wie möglich zu halten.

Bei der fast-DIXON Methode handelt es sich um eine Messung nach der DIXON Methode und unter Verwendung eines bipolaren Auslesegradientens. Es ist eine schnelle Spin-Echo (fast spin-echo, FSE) Messung.

Die fast-DIXON Methode ist bekannt und eine Übersicht zur Durchführung sowie der Vor- und Nachteile dieses Verfahrens werden von Jingfei Ma et al. in "Fast Spin-Echo Triple-Echo Dixon (fTED) Technique for Efficient T2-Weighted Water and Fat Imaging" (Magnetic Resonance in Medicine 58; 103-109 (2007)) und den darin zitierten Quellen beschrieben.

Der vorliegenden Erfindung liegt die Aufgabe zugrunde, ein Verfahren bereitzustellen, bei dem die Informationen, die nach einer MR-Messung nach der DIXON Methode unter Verwendung eines bipolaren Auslesegradientens (fast-DIXON) erhalten werden, gleiche bzw. vergleichbare Qualität aufweisen, wie sie durch eine MR-Messung nach der DIXON Methode mit monopolaren Auslesegradienten erhalten werden. Dabei soll die MR-Aufnahmezeit zumindest nicht erhöht werden.

Diese Aufgabe wird jeweils durch einen Gegenstand nach den unabhängigen Ansprüchen 1, 11 und 13 gelöst. Vorteilhafte Ausführungsformen sind Gegenstand der abhängigen Ansprüche, der Beschreibung und der Zeichnungen.

Im Besonderen bezieht sich die vorliegende Erfindung auf ein auf Deep-Learning basierendes Korrekturverfahren, durch das chemische Verschiebungs-Artefakte, CSA, aus den MR-Aufnahmedaten entfernt werden. Dieses Verfahren ist in Anspruch 1 definiert. Des weiteren bezieht sich die vorliegende Erfindung auf ein System zur Ausführung des Verfahrens gemäß Anspruch 11 sowie ein Computerprogramm gemäß Anspruch 13.

Der technische Vorteil ist in der beschleunigten Bilddatenerfassung und in einer dabei deutlich verbesserten Bildqualität zu sehen, was sich ebenfalls vorteilhaft auf die Folgeprozesse, wie z.B. medizinische Auswertungen und Diagnosen auswirkt.

Der Begriff "Richtung" bezieht sich auf die Ausleserichtung.

So treten bei der TSE-Sequenz CSA Artefakte nur in Ausleserichtung auf. Die Ausleserichtung der Gradienten kann auch als Aufnahmerichtung verstanden werden.

Die Begriffe "gleich- und gegenphasig" beziehen sich auf die Phasenlage der Wasser- und Fettspins zueinander. Die Phasenlage (also gleich bzw. in-phase oder gegenphasig bzw. opposed-phase) kann unabhängig von der Polarität behandelt werden. So hat die Phasenlage z.B. mit "monopolar" (Auslesegradient für gleich- und gegenphasiges Echo in gleicher Richtung) und "bipolar" (Auslesegradienten in unterschiedlichen Richtungen) erstmal nichts zu tun. Die zweite Art erhält man mit einer monopolaren Aufnahme die erste Art mit einer bipolaren Aufnahme.

Zum Training des Netzwerkes kann man in- und opposed-Daten aus einer monopolaren Aufnahme wie beschrieben durch einen Dixon-Algorithmus schicken und erhält dann Fett- und Wasserbilder. Durch Verschieben der Bilder gegeneinander in jeweils beide Richtungen kann man wieder bipolare Aufnahmen und damit Trainingsdaten simulieren.

Die Eingangsdaten des Netzwerkes sind Bilder mit CSA in unterschiedliche Richtungen. Ausgangsdaten sollen Bilder mit CSA in gleiche Richtung sein.

In einer bevorzugten Ausführungsform der Erfindung wird Schritt (2) nach Schritt (1) ausgeführt. Alternativ ist es möglich, dass man die Abfolge der Schritte variiert. So kann es z.B. Sinn machen, Schritt (2) vor Schritt (1) auszuführen, indem z.B. "alte" Daten in die maschinelle Lernstruktur oder das CNN geladen werden.

Bei den Aufnahmen handelt es sich um solche, bei denen Wasser- und Fettspins in Phase sowie außer Phase sind. Die CSA treten an den Grenzflächen zwischen Wasser und Fett auf. Bei den Aufnahmen sind Fett und Wasser allerdings nicht bereits getrennt aufgenommen (obwohl es solche Verfahren auch gibt; hierbei funktioniert allerdings die Unterdrückung der jeweils anderen Spezies oftmals nicht immer in ausreichender Qualität; daher ist das DIXON-Verfahren überhaupt erst notwendig).

In einer weiteren bevorzugten Ausführungsform der Erfindung handelt es sich bei den Aufnahmedaten in Schritt (1) und/oder insbesondere bei den Trainingsdaten und bei den Inputdaten des CNN um Bild-Rohdaten in einem k-Raum. Alternativ oder kumulativ kann es sich um rekonstruierte Bilder im Bildraum handeln. Alternativ kann das Verfahren auch in einem Hybrid-Modus angewendet werden, so dass es sich bei den Aufnahmedaten (Trainingsdaten und CNN-Inputdaten) um hybride Bilddaten handelt. "Hybride Bilddaten" sind Daten, die teils im k-Raum und teils im Bildraum repräsentiert sind. Hybride Bilddaten sind insbesondere Daten, bei denen eine FourierTransformation vom k-Raum in den Bildraum nur in einer Raumrichtung, vorzugsweise der Ausleserichtung, durchgeführt wurde.

Der Output des CNN sind in- und gegenphasige-Daten, die anschließend einem Dixon-Algorithmus zugeführt werden. Es ist auch beispielsweise möglich, in einem nicht zur Erfindung gehörenden Verfahren, den Dixon-Algorithmus direkt in das CNN zu integrieren, so dass der Output des CNN direkt die rekonstruierten Bilddaten sind (ohne CSA). Derartige CNNs zeigen beispielsweise die Schriften "Magnitude and Complex Single- and Multi-echo Water Fat Separation via End-to-End Deep Learning", Goldfarb JW, Proc. Int. Soc. Magn. Res. in Med., ISMRM, Paris, France, 16.-21.06.2018, Nr.

5608, 1. Juni 2018 und "Deep Neural Network for Single-Point Dixon Imaging with Flexible Echo Time", Son JB et al., Proc. Int. Soc. Magn. Res. in Med., ISMRM, 11.-16.05.2019, Montreal, Canada, Nr. 4014, 26. April 2019.

In einer weiteren bevorzugten Ausführungsform der Erfindung weist das CNN eine U-Net-Architektur oder eine GAN-Architektur auf.

Das U-Net ist eine Faltungsnetzarchitektur, das von Wissenschaftlern der Uni Freiburg für die schnelle und präzise Segmentierung von Bildern entwickelt worden ist. Grundsätzlich erfordert ein erfolgreiches Training von tiefen Netzwerken viele tausend annotierte Trainingsmuster. Das U-Netzwerk und die dazugehörige Trainingsstrategie beruht auf dem starken Einsatz von Datenerweiterung, um die verfügbaren annotierten Eingangsdaten effizienter zu nutzen. Die Architektur besteht aus einem kontrahierenden Pfad zur Erfassung des Kontexts und einem symmetrischen expandierenden Pfad, der eine präzise Lokalisierung ermöglicht. Es wurde gezeigt, dass ein solches Netzwerk von Ende zu Ende aus sehr wenigen Bildern trainiert werden kann und die bisher beste Methode (ein Sliding-Window-Faltungsnetzwerk) übertrifft. Außerdem ist das Netzwerk schnell. Die Segmentierung eines 512x512 Bildes dauert weniger als eine Sekunde auf einer aktuellen GPU. Die vollständige Implementierung (basierend auf Caffe) und die trainierten Netzwerke sind unter http://lmb.informatik.uni-freiburg.de/people/ronneber/u-net verfügbar. Für technische Details sei auf die folgende Publikation verwiesen: http://lmb.informatik.uni-freiburg.de/Publications/2015/RFB15a. Mittlerweile wurde die U-Net-Architektur auch auf Bildrekonstruktionen und - transformationen erweitert (vgl. z.B. https://www.ncbi.nlm.nih.gov/pmc/articles/PMC6785508/pdf/qims -09-09-1516.pdf)

Die CNN-Architekturen sind in der Regel so konzipiert, dass sie mit ausgewogenen Datensätzen gut funktionieren. Ein häufig auftretendes Problem bei realen Datensätzen ist, dass sie eben nicht ausgewogen sind und unter einem Ungleichgewicht der Klassen leiden, so dass also z.B. nur eine kleine Anzahl von Artefaktbildern zum Trainieren der CNNs zur Verfügung, wohingegen viele Gutbilder (ohne Artefakt) vorhanden sind oder umgekehrt. Dies führt zu dem Problem, dass die Netze allein auf Grund der Unausgewogenheit der Daten dann dazu neigen, ein Bild einseitig und entsprechend der Unausgewogenheit zu klassifizieren, da dieser Vorgang im Trainingsprozess meist zu einer korrekten Klassifizierung führte und somit mit einem kleinen Loss belohnt wurde. Es sind verschiedene Methoden bekannt, dieses Ungleichgewicht zu reduzieren oder zu kompensieren. Eine häufig verwendete Methode ist die Datenaugmentierung. Dabei werden leicht modifizierte Kopien von bereits vorhandenen Daten erstellt und zu der entsprechenden Klasse hinzugefügt. Eine weitere Möglichkeit ist die Anpassung der Gewichtung. In diesem Fall werden Fehlklassifikationen von Instanzen aus Klassen mit wenig Daten stärker gewichtet.

Im Gegensatz zu den genannten Ansätzen zielen Generative Adversarial Neural Networks (GANs) in diesem Kontext darauf ab, die zugrundeliegenden Datenverteilungen aus den begrenzten verfügbaren Bildern zu lernen und dann die gelernten Verteilungen zu verwenden, um synthetische Bilder zu erzeugen. GANs können verwendet werden, um synthetische Bilder für Klassen mit wenig Bilddaten verschiedener unausgeglichener Datensätze zu erzeugen. Sie könnten somit als eine intelligente Over-sampling-Methode verwendet werden. GANs sind nicht nur in der Lage ein synthetisches Bild zu generieren, sondern bieten auch eine Möglichkeit, etwas am Originalbild zu verändern.

GANs bestehen aus zwei künstlichen neuronalen Netzen. Ein Netz ist der sogenannte Generator, der aus einem Vektor latenter Variablen künstliche Daten erzeugt. Das zweite Netz ist der Diskriminator, der die Daten auswertet und versucht, zwischen künstlichen und realen Daten zu unterscheiden. Diese beiden Netzwerke arbeiten sozusagen "gegeneinander" und führen im Trainingsprozess ein Nullsummenspiel durch. Der Generator versucht, Daten zu erzeugen, die den realen Daten so ähnlich sind, dass der Diskriminator nicht in der Lage ist, die künstlich erzeugten Daten von den realen Daten zu unterscheiden. Die ersten GAN-Architekturen benötigten eine große Menge an Daten für die Trainingsphase und konnten nur Bilder mit begrenzter Auflösung erzeugen. Neuere Architekturen wie SinGAN7 oder StyleGAN28, die mit Adaptive Discriminative Augmentation (ADA) arbeiten, erlauben es jedoch, auch mit kleinen Datensätzen hochwertige Bilder zu erzeugen.

Für die Anwendung von GANs und anderen Architekturen in der Medizin sei auf den Artikel verwiesen: https://www.mdpi.com/2076-3417/10/5/1816/pdf.

In einer weiteren bevorzugten Ausführungsform der Erfindung umfasst das Anwenden des trainierten CNN das Bereitstellen von nxm-kanaligen Bilddaten in eine Eingabeschicht des CNN, wobei n die Anzahl der Ausleseschritte und m die Anzahl der Phasenkodierschritte ist. Insbesondere kann ein 4-Kanal Bild oder ein 6-Kanal Bild mit z.B. 256x256 Pixeln als Input verwendet werden.

In einer weiteren bevorzugten Ausführungsform der Erfindung kann ein 3-Punkt-Dixon Verfahren zur Anwendung kommen. In diesem Fall sind die Bilddaten dann 6-kanalig usw. Das bringt Vorteile für die Performance des Netzes mit sich.

In einer weiteren bevorzugten Ausführungsform der Erfindung umfassen die Inputdaten reale und imaginäre Anteile der aus den gleichphasigen und gegenphasigen Echos erzeugten Aufnahmedaten, die miteinander konkateniert sind. Die Begriffe "realer/imaginärer Anteil" beziehen sich auf den Umstand, dass die MR-Signale komplexwertig sind. Bei der Verarbeitung in neuronalen Netzen werden diese in aller Regel nach Real- und Imaginärteil (nicht Magnitude und Phase) aufgeteilt. "Konkateniert" meint in diesem Kontext, eine Verkettung zweier Tensoren entlang einer Dimension.

Die Konkatenation der Input-Daten dient dazu, zusammengehörige Daten zusammenzuhalten oder auch zusammen zu speichern. Bei echten Bildaufnahmen könnten das z.B. die RGB-Kanäle sein.

Die Verkettung im extrahierenden Zweig mit Daten aus dem kontrahierenden Zweig hat den Zweck, die Auflösung wiederherstellen zu können. Eine Interpolation von ganz kleiner auf ganz große Matrix wäre sonst instabil. Deshalb fließen die Tensoren mit der gleichen Auflösung aus dem Kontraktionszweigs mit ein. Üblicherweise wird auf jeder Auflösungsstufe verkettet.

In einer weiteren bevorzugten Ausführungsform der Erfindung erfolgt das Training mit monopolaren Aufnahmedaten und somit ohne Zuordnung von bipolaren Aufnahmedaten. Das Lernverfahren kann als unüberwachtes Lernen ausgebildet. Aus den monopolaren Aufnahmedaten können mittels eines Schätzalgorithmus bipolare gleichphasige und gegenphasige Aufnahmedaten erzeugt (geschätzt) werden. Der Schätzalgorithmus kann in einer weiteren Ausführungsform der Erfindung ein Rauschen hinzufügen, um Aufnahmedaten mit einer größeren Bandbreite zu simulieren, wobei die Bandbreite Einfluss auf die Position der chemischen Verschiebung hat.

In einer weiteren bevorzugten Ausführungsform der Erfindung werden die Aufnahmedaten aus gegenphasigen Echos erzeugt, die in um einen von 180° abweichenden Phasenwinkel verschoben sind, indem die Daten der Fettprotonen mit $e^{(i*\text{Phasenwinkel})}$ multipliziert werden, wobei der Phasenwinkel die tatsächliche Dephasierung im Bereich von 0 bis 2pi ist.

Das Verwenden des Magnetresonanz-DIXON Verfahrens mit bipolaren Auslesegradienten, nachfolgend "fast-DIXON-MR" oder "bipolares DIXON-MR" genannt, hat gegenüber dem Magnetresonanz-Dixon Verfahrens mit monopolaren Auslesegradienten, nachfolgend "DIXON-MR" oder "monopolares DIXON-MR" genannt, den Vorteil, dass die Aufnahmezeit mindestens halbiert wird und es zu minimalen störenden Bewegungseffekten bei der Aufnahme kommt. MRT Untersuchungen werden oft an Patienten vorgenommen und die Verkürzung der Aufnahmezeit trägt maßgeblich zur Akzeptanz dieses Verfahrens bei.

Bei der Aufnahme von bipolaren DIXON-MR Aufnahmedaten werden eine Vielzahl von K-Raum-Echos, vorzugsweise bidirektional oder in unterschiedlichen Raumrichtungen erfasst, wobei mindestens ein Echo ein gleichphasigen (gerades) Echo ist, das in einer ersten Richtung erfasst wird, und mindestens ein Echo ein gegenphasigen (ungerades) Echo ist, das in einer zweiten Richtung, z.B. entgegengesetzt zur ersten Richtung erfasst wird. Die K-Raum-Echoausrichtung wird zwischen dem geraden und dem ungeraden Echo korrigiert.

Das fast-DIXON-MR Verfahren stellt ein Zwei- oder Mehrpunktverfahren dar, wobei Zweipunkt bedeutet, dass jeweils die Gleichphase (auch "in-phase" genannt) und die Gegenphase (auch "opposed-phase" genannt) gemessen werden. Diese Messung läßt sich auf mehre Phasen ausweiten, z.B. auf drei Phasen in der Abfolge Messung der Gegenphase, Gleichphase, Gegenphase.

Unter dem Begriff "Artefakt" sind vorliegend nur Artefakte gemeint, die dem Verfahren der MRT zugerechnet werden müssen, nicht jedoch solche, die aufgrund einer fehlerhaften Auswahl der verschiedenen Parameter auftreten. Artefakte, die dem Verfahren der MRT zuzurechnen sind, sind zum Beispiel Bewegungs-Artefakte und chemischen Verschiebung-Artefakte (auch "chemical shift artefact" oder "CSA" genannt).

Das Auftreten solcher chemischen Verschiebungs-Artefakte ist bei einer MR-Messung nach der fast-DIXON Methode ausgeprägt vorhanden, da die Echos mit unterschiedlichen Polaritäten des Auslesegradienten aufgenommen werden.

Als "chemische Verschiebung" bezeichnet man die Eigenschaft, dass sich die Resonanzfrequenz abhängig von der Art der chemischen Bindung, in der sich ein signalgebender Kern befindet, proportional zur Feldstärke geringfügig verschiebt. Aufgrund ihrer Konzentration im menschlichen Körper tragen hauptsächlich Wasserstoffkerne des freien Wassers und des Fettes zum Bild bei. Deren relative Resonanzfrequenzdifferenz beträgt etwa 3 ppm (parts per million).

Dadurch kommt es bei der Verwendung von Spinecho- sowie Gradientenechosequenzen zu einer Modulation der Signalintensitäten in Abhängigkeit von der Echozeit.

Der CSA entsteht an den Grenzflächen zwischen Wasser und Fett und wird durch die unterschiedlichen Resonanzfrequenzen verursacht. Aufgrund der leicht unterschiedlichen Frequenzen der Spins von Wasser- und Fettprotonen können im MR-Bild die Positionen der unterschiedlich gebunden Protonen in Richtung des Auslesegradienten gegeneinander verschoben werden. Nach der Dixon-Rekonstruktion sind die Grenzbereiche im Wasserbild scharf abgegrenzt und im Fettbild sind diese Grenzen unscharf. Bei den in- und opposed phase-Bildern sind die Artefakte wie oben beschrieben nur auf der jeweils anderen Seite der Grenzfläche zu finden.

Unter Qualität wird vorliegend verstanden, dass die Information in den entsprechenden Bildern eindeutige Aussagen über die aufgenommenen Strukturen zulassen.

Unter gleichphasig wird vorliegend verstanden, dass die Magnetisierungsvektoren von Wasser (W) und von Fett (F) parallel sind und in die gleiche Richtung zeigen (W+F) und unter gegenphasig wird verstanden, dass diese Magnetisierungsvektoren parallel sind, jedoch in die entgegengesetzte Richtung (W-F) zeigen.

Die Erfindung bezieht sich auf den Einsatz eines oder mehrerer neuronalen Netzwerks/e, insbesondere eines CNN (convolutional neural network), um bipolare DIXON-MR Aufnahmedaten so zu verändern, dass sie mit monopolaren DIXON-MR Aufnahmedaten übereinstimmen und somit minimale, vorzugsweise keine, CSA in verschiedenen Raumrichtungen aufweisen.

Der Vorteil der schnelleren Aufnahmezeit der fast-DIXON Methode kann somit vorteilhafterweise genutzt werden ohne die Qualität der Aufnahmedaten zu schmälern.

In einer bevorzugten Ausführungsform der Erfindung wird eine U-Net Netzwerkarchitektur verwendet. Nach den Faltungsschichten (convolutional layers) erfolgt eine Verbindung zwischen einem Kontraktionspfad bzw. Down-Samplingpfad und einem Expansionspfad bzw. Up-Samplingpfad des Netzwerkes, was die Propagierung der ursprünglichen Daten in die Up-Sampling-Pfade erlaubt.

In einer alternativen vorteilhaften Ausführungsform der Erfindung wird statt des U-Net eine andere Netzwerkarchitektur, wie z.B. ein GAN (generative adversarial network) Netzwerk verwendet.

Die Eingangsschicht (Input-Layer) besteht aus den Real- und Imaginärteilen der gleichphasigen und gegenphasigen Daten der bipolaren DIXON-MR Aufnahme in einer Vierkanaldarstellung. Die Verkettung bzw. Konkatenation ist notwendig, um eine Abschätzung der nicht-verschobenen Bilder von den beiden Datensätzen (Gleich- und Gegenphase), mit Verschiebungen in die entgegengesetzte Richtung, zu erhalten.

Die Ausgabeschicht (output layer) hat die gleiche Dimensionalität und enthält gleich- und gegenphasige Daten mit reduzierten oder eliminierten chemischen Verschiebungen.

Da chemische Verschiebungen in Lesekodierungsrichtung auftreten, ist es bevorzugt, diese Dimension im Bildraum zu propagieren, d. h. entweder die vollständige Bildraumdarstellung (x,y) oder eine Hybridraumdarstellung (x, ky) zu verwenden. In einer alternativen Ausführungsform der Erfindung kann auch eine reine k-Raum Repräsentation der Daten (kx, ky) als Eingangsdaten (sowohl für das Training als auch in der Inferenzphase) verwendet werden.

In einer anderen vorteilhaften Ausführungsform der Erfindung ist es auch möglich, Wasser- und Fettbilder nach der DIXON Rekonstruktion anstelle von gleich- und gegenphasigen Bildern zu verwenden. Da diese Bilder bereits eine nichtlineare Mischung aus gleich- und gegenphasigen Bildern enthalten, könnten die Ergebnisse mit diesem Ansatz allerdings schlechter sein.

In einer anderen nicht zur Erfindung gehörenden Ausführungsform enthält die Eingabeschicht die gleich- und gegenphasigen Bilder und die Ausgabeschicht enthält Fett- und Wasserbilder und optional eine Phasenkarte (field map), die aus den Eingabedaten entfernt wurde, d. h. eine Einbeziehung der DIXON Rekonstruktion in das neuronale Netz.

Zum Training des Netzes können bipolar erfasste gleich- und gegenphasige Daten (z.B. TSE-DIXON mit der Option fast-DIXON-MR) als Eingangsdaten und monopolare gleich- und gegenphasige Daten (z.B. TSE-DIXON ohne fast-DIXON-MR) als Zieldaten verwendet werden. Es kann ein überwachtes Lernverfahren zur Anwendung kommen. In einer Implementierung, die auf der PyTorch Plattform basiert, kann vorzugsweise als Netzarchitektur ein U-Net bestimmt werden, um, dann die Tensoren mit allen Eingangs- und Ausgangsdaten zu bilden, und die Daten dann in Trainings- und Validierungsdaten aufteilen zu lassen und dann das Netz trainieren. Das Netz kann dann z.B. mittels Backpropagation anhand einer Metrik (z.B. L2-Norm zwischen erwünschtem Zielbild und vom Netz generierten Bild) trainiert werden.

Eine alternative bevorzugte Ausführungsform der Erfindung erfordert keine separat gelabelten Daten (mit zugeordneten Zieldaten) als Trainingsdaten, sondern kann die Trainingsdaten selbst erzeugen. So kann beispielsweise aus jeden monopolaren DIXON-MR Aufnahmedaten eine große Anzahl von bipolaren Dixon-MR Aufnahmedaten als Trainingsdatensätzen gewonnen werden. Der Algorithmus kann eine Zuordnung vorsehen, welches der aus dem Dixon-Algorithmus rekonstruierten Bildern Fett bzw. Wasser enthält.

Erstens werden mehrere chemische Verschiebungen simuliert, indem verschiedene (Sub-) Pixelverschiebungen mit jeweils unterschiedlichen Vorzeichen auf Fettbilder in Ausleserichtung angewendet werden. Dies hat den technischen Hintergrund, um das bipolare Auslesen jeweils einer Aufnahme zu simulieren.

Zweitens wird eine Phasenkarte entweder aus der konventionellen DIXON Rekonstruktion oder einem entsprechenden Pre-Scan entnommen oder aus den Bildern synthetisiert.

Aus dieser Phasenkarte wird eine Vielzahl von Phasenkarten erzeugt, z. B. durch Addition von Offsets, Multiplikation mit Skalaren oder Überlagerung von einer Eigenfunktion, insbesondere einer Kugelflächenfunktion.

Die Fettbilder werden dann zum Wasserbild addiert und vom Wasserbild subtrahiert mit anschließender Multiplikation des komplexen Exponentials der synthetisierten oder künstlichen Feldkarten, um synthetisierte oder künstliche bipolare Aufnahmedaten zu erzeugen.

Außerdem können alle resultierenden Bilder mit zusätzlichen Phasenkarten weiter multipliziert werden. Zusätzlich kann künstliches Rauschen injiziert werden, um Aufnahmedaten mit höherer Bandbreite zu simulieren.

Darüber hinaus können phasenverschobene Daten mit einem anderen Phasenwinkel als 180° für den gegenphasigen Fall durch eine Multiplikation der Fettdaten mit e^(i*Phasewinkel) erzeugt werden, wobei der Winkel die tatsächliche Dephasierung im Bereich von 0 bis 2pi ist.

Zielbilder für das Training erhält man, indem man wie beschrieben vorgeht, aber 1. den Schritt der Pixelverschiebung weglässt, 2. weniger Rauschen einspeist und 3. wirbelstrombedingten Effekte auf die Phasenkarte (Fieldmap) weglässt. Letzteres könnte durch Subtraktion einer mit konventionellen Ansätzen (d. h.

einer 2- oder 3-Echo-Gradientenecho-Akquisition) erhaltenen Feldkarte von der mit dem DIXON Algorithmus auf einem bipolaren Datensatz erhaltenen Feldkarte erfolgen.

Vorzugsweise beträgt die Phasenverschiebung (Dephasierung) 180°. In diesem Fall wirken die Vektoren genau entgegengesetzt und die Signale heben sich auf. Alternative Ausführungsformen sehen eine von 180° abweichende Phasenverschiebung vor. Das Netzwerk muss dann entsprechend mit Daten dieser Phasenverschiebung trainiert worden sein.

Vorstehend wurde die Lösung der Aufgabe anhand des Verfahrens beschrieben. Dabei erwähnte Merkmale, Vorteile oder alternative Ausführungsformen sind ebenso auch auf die anderen beanspruchten Gegenstände zu übertragen und umgekehrt. Mit anderen Worten können auch die auf ein System oder auf ein Computerprogrammprodukt gerichteten unabhängigen Ansprüche mit den Merkmalen weitergebildet sein, die in Zusammenhang mit dem Verfahren beschrieben oder beansprucht sind. Die entsprechenden funktionalen Merkmale des Verfahrens werden dabei durch entsprechende gegenständliche Module, insbesondere durch Hardware-Module oder Mikroprozessor-Module, des Systems bzw. des Produktes ausgebildet und umgekehrt.

Gemäß einem weiteren Aspekt bezieht sich die Erfindung auf ein System gemäß Anspruch 11 sowie ein Computerprogramm gemäß Anspruch 13.

In der folgenden detaillierten Figurenbeschreibung werden nicht einschränkend zu verstehende Ausführungsbeispiele mit deren Merkmalen und weiteren Vorteilen anhand der Zeichnung besprochen. In dieser zeigen:

Fig. 1     ein Sequenzdiagramm einer konventionellen bipolaren TSE Dixon (Fast DIXON) Akquisition;

Fig. 2     Beispielaufnahmen einer monopolaren DIXON-MR Akquisition und einer bipolaren fast-DIXON MR Akquisition;

Fig. 3     ein Blockschaltbild gemäß einer bevorzugten Ausführungsform der Erfindung einer U-Net Architektur zur CSA Korrektur, die in fast-DIXON Verfahren, welche bipolare Auslesegradienten zur Erfassung der gleich- und gegenphasigen Echos verwenden, ausgeprägt vorhanden sind;

Fig. 4     eine Übersichtsdarstellung für ein Training des Netzwerkes gemäß einer bevorzugten Ausführungsform der Erfindung;

Fig. 5     ein Ablaufdiagramm eines Verfahrens gemäß einer bevorzugten Ausführungsform der Erfindung; und

Fig. 6    ein Blockschaltbild von Modulen eines Magnetresonanz-Bildgebungsgerätes.

Detaillierte Beschreibung der Figuren

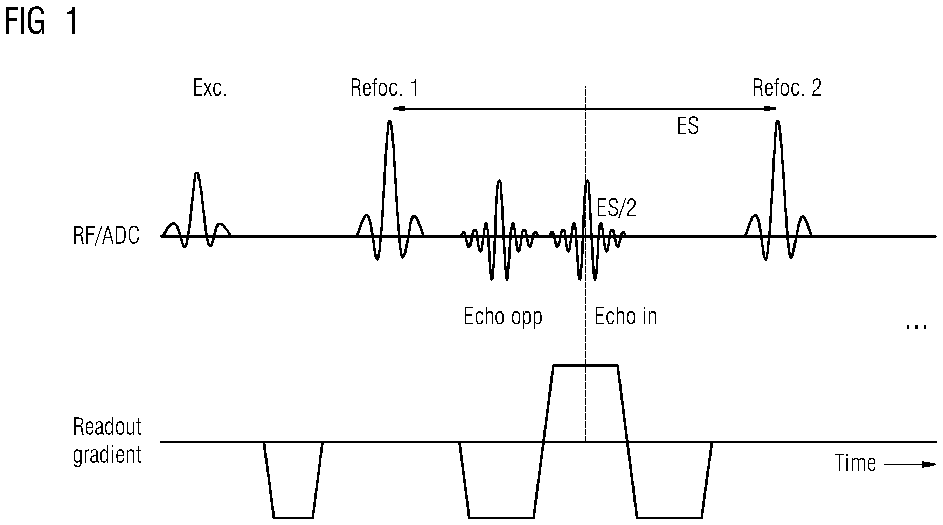

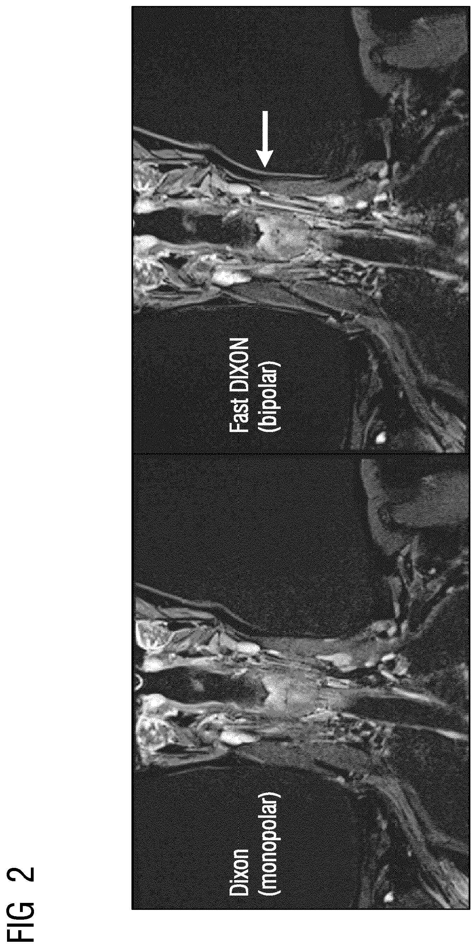

Fig. 1 zeigt ein typisches Sequenzdiagramm einer konventionellen fast Dixon Aufnahme. Bei der Dixon-Bildgebung erfolgt die Datenerfassung zu mindestens zwei verschiedenen Zeitpunkten, so dass das Signal von Wasser- und Fett-Spins in Phase bzw. außer Phase (gegenphasig) ist. Ein Nachbearbeitungsalgorithmus kann dann das Fett- und Wassersignal trennen und zwei Bilder erzeugen, die nur den Fett- bzw. Wasseranteil darstellen. Um die Erfassung zu beschleunigen, werden Ein- und Gegenphasenechos oft bipolar erfasst. Dies wird z. B. bei der VIBE-Bildgebung und mit der Fast DIXON-Funktion für TSE durchgeführt, bei der die Erfassung eines In-Phase- und eines Gegenphasenechos nach einem Refokussierungsimpuls erfolgt (siehe Figur 1 mit "Refoc" bezeichnet). Das Intervall zwischen einem In-Phase- und einem Out-of-Phase-Zustand nimmt mit zunehmender Feldstärke linear ab. Bei 3 T beträgt dieses Intervall nur noch etwa 1,2 ms. Ein- und Gegenphasenechos werden mit einem bipolaren Gradienten ausgelesen. Da die Position der chemischen Verschiebungsartefakte von der Polarität des Gradienten abhängt (vgl. Figur 2), treten die Artefakte bei den Gegenphasen- und Einphasen-Echos an unterschiedlichen räumlichen Positionen auf. (In Fig. 1 ist nur der erste Teil der Echokette abgebildet).

Für konventionelles monopolares DIXON gilt:

IP = W($\delta$) + F($\delta$+chs), wobei IP gleichphasig (in phase) kennzeichnet und W für Wassser und F für Fett steht;
OP = W($\delta$) - F($\delta$+chs), wobei OP gegenläufiger Phase (opposed phase) kennzeichnet.

Für bipolares DIXON gilt:

$$IP = W(\delta) + F(\delta+chs)$$

$$OP = W(\delta) - F(\delta-chs)$$

Hier sind IP und OP die in- bzw. gegenphasigen Datensätze, W ist das Wassersignal, F ist das Fettsignal, $\delta$ ist die Voxelposition und chs ist die chemische Verschiebung, die durch den Unterschied zwischen den Larmor-Frequenzen von Wasser- und Fettprotonen eingeführt wird.

Durch Addition und Subtraktion von Ein- und Gegenphasenbildern können die Fett- und Wasserbilder für die konventionelle DIXON-Bildgebung erhalten werden:

$$0,5 * (IP+OP) = W(\delta)$$

$$0,5 * (IP-OP) = F(\delta+chs)$$

Hier tritt die chemische Verschiebung als globale Verschiebung für alle Voxel nur im Fettbild auf. Fett- und Wasserbilder können dann, falls gewünscht, durch einfache räumliche Registrierung ausgerichtet werden.

Fig. 2 zeigt in einem Beispiel die Qualitätsunterschiede bei einer monopolaren und bipolaren Dixon Aufnahme. Der chemische Verschiebungsartefakt, chemical shift artifact, CSA, wird durch unterschiedliche Larmor-Frequenzen für Fett- und Wasserprotonen verursacht und ist in den Aufnahmen als Linienartefakt zu sehen. Wenn der Auslesegradient von links nach rechts zunimmt, wird Fett nach links verschoben, wenn der Auslesegradient von links nach rechts abnimmt, wird Fett nach rechts verschoben. Auf der rechten Seite ist die Auswirkung der unterschiedlichen chemischen Verschiebung auf die endgültigen Fett- und Wasserbilder dargestellt. Besonders bei den Wasserbildern treten an den Fett-Wasser-Grenzen Ringing-Artefakte auf. (Quelle: Leinhard et al, ISMRM 2008).

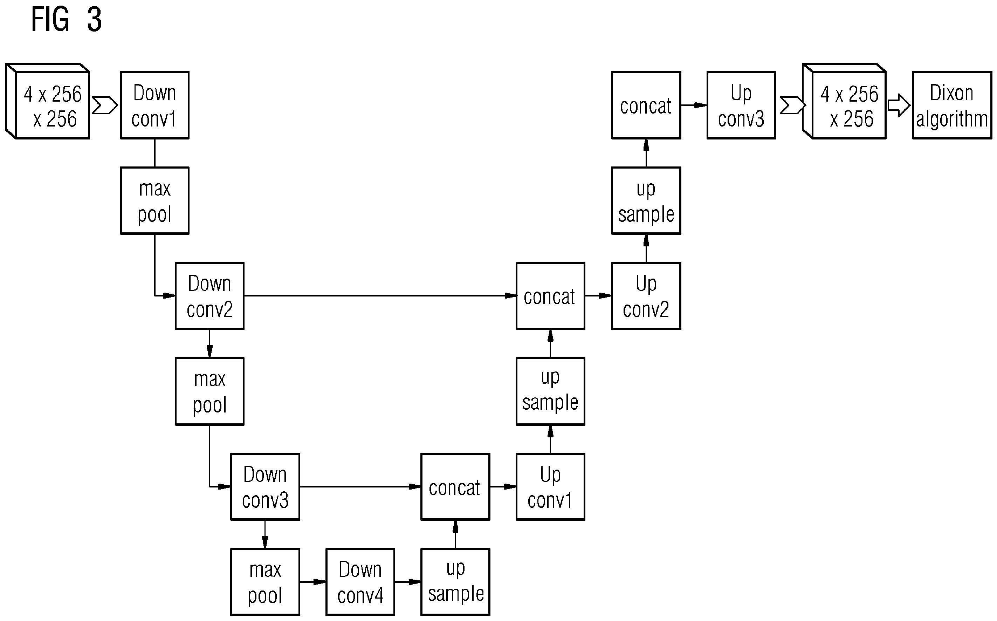

Eine mögliche Netzwerkarchitektur ist in Fig. 3 dargestellt. Hier wird eine U-Netz-Architektur mit übersprungenen Verbindungen gewählt, die die Propagierung der Originaldaten in den Upsampling-Pfad des Netzwerks ermöglicht.

Die Eingangs- und Ausgangsdaten bestehen aus Real- und Imaginärteilen von Ein- und Gegenphasendaten, die in der Kanaldimension verkettet werden und ein 4-Kanal-Bild ergeben. Es kann eine im Stand der Technik bekannte CNN-Architektur verwendet werden, wie sie von Ronneberger et al entwickelt worden ist (vgl. Ronneberger, Olaf; Fischer, Philipp; Brox, Thomas (2015). "U-Net: Convolutional Networks for Biomedical Image Segmentation". arXiv:1505.04597). Nach dem Upsampling werden die 4-Kanal-Daten wieder in Ein- und Gegenphasenbilder aufgeteilt und in einen konventionellen Dixon-Algorithmus eingespeist, um in einem letzten Schritt Fett- und Wasserbilder zu erhalten.

Die Eingabeschicht besteht aus den Real- und Imaginärteilen der Ein- und Gegenphasendaten der bipolaren Dixon-Aufnahmen in einer 4-Kanal-Darstellung. Die Verkettung ist notwendig, um die Schätzung der nicht verschobenen Bilder aus den beiden Datensätzen mit Verschiebungen in entgegengesetzter Richtung zu ermöglichen.

Anstelle eines U-Netzes könnten auch andere Netzwerkarchitekturen wie z. B. ein Generatives Adversariales Netzwerk (GAN) verwendet werden.

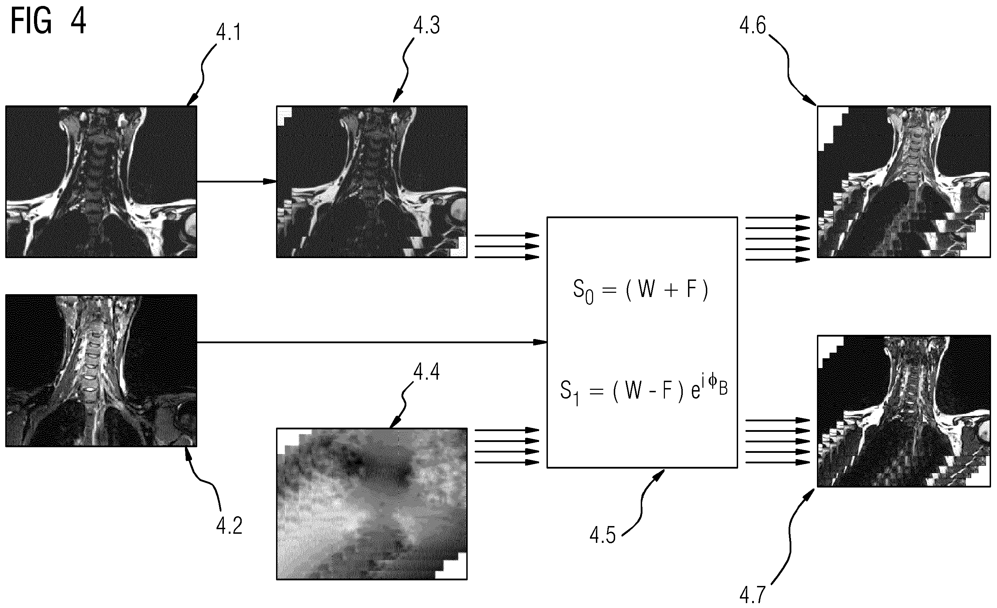

Fig. 4 zeigt anhand von Beispielbildern einen Trainingsansatz zum Training des Netzwerks, der die Erstellung einer Vielzahl von Trainingsdatensätzen aus einer einzigen monopolaren Dixon-Rekonstruktion ermöglicht.

Mit dem Bezugszeichen 4.1 ist die monopolare Fettaufnahme (Fettbilddaten) und mit 4.2 die monopolare Wasseraufnahme (Wasserbilddaten) gekennzeichnet. Das Bezugszeichen 4.3 repräsentiert die Anwendung einer Vielzahl von Pixelverschiebungen entlang der Ausleserichtung, um den CSA mit unterschiedlichen Bandbreiten zu simulieren. Bezugszeichen 4.4 kennzeichnet die Verwendung der Phasenkarte (fieldmap) $\Phi_B$ aus der Dixon Rekonstruktion. Optional können noch diverse weitere geänderte Phasenkarten erzeugt werden. Bei 4.5. wird eine Vielzahl von künstlichen gleich- und gegenphasigen Bildern erzeugt, gemäß der Gleichung, wie in Fig. 4 dargestellt. Daraus können dann in 4.6 dargestellten simulierten bipolaren gleichphasigen Bilder und die in 4.7 dargestellten simulierten bipolaren gegenphasigen Bilder erzeugt werden.

Die vorgeschlagene Methode ermöglicht die schnelle Erfassung von bipolaren Dixon-Daten mit verbesserter Bildqualität, da die chemische Verschiebung in verschiedene Richtungen vor der Dixon-Rekonstruktion entfernt wird, was zu weniger Unschärfe führt. Darüber hinaus können auch Ring-Artefakte (ringing artifact; entstehend durch die Fouriertransformations-basierten bildverarbeitenden Berechnungen einer abgetasteten Signalzeile) verringert werden.

Neben der Dixon-Bildgebung kann die Methode auch für andere bipolare Erfassungsschemata wie Mapping-Sequenzen oder TGSE-Sequenzen eingesetzt werden.

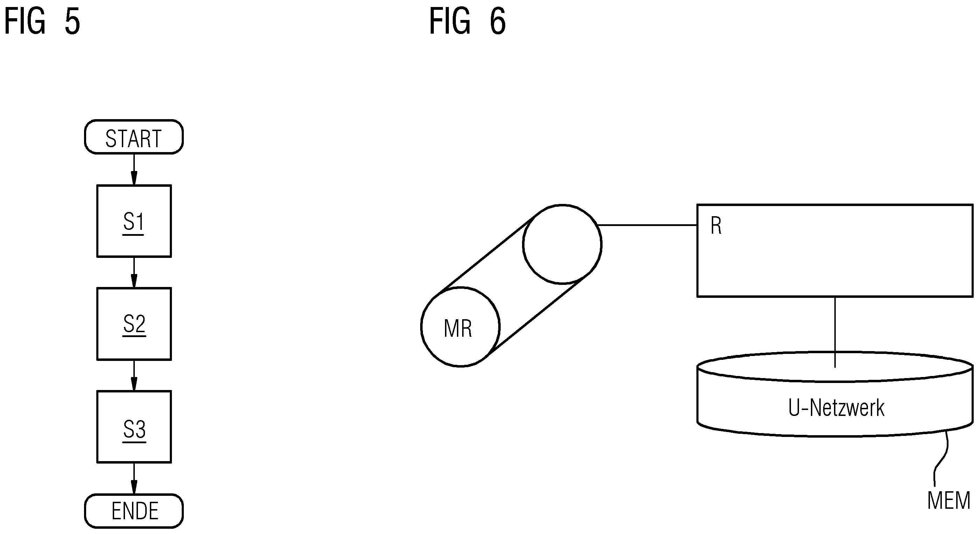

Fig. 5 ist ein Ablaufdiagramm des Verfahrens gemäß einer bevorzugten Ausführungsform der Erfindung. Nach dem Start wird in Schritt S1 das trainierte CNN bereitgestellt. In einer Vorverarbeitung kann das Netzwerk trainiert worden sein. Dazu können Trainingsdaten auf synthetische Weise erzeugt worden sein, indem z.B. ein Teil der Bilder, z.B. die CSA enthaltenden Bilder mit CSA in unterschiedlichen Richtungen, aus vorliegenden Bilddaten durch Bildverarbeitungsprozeduren erzeugt worden sind. In Schritt S2 werden zumindest zwei bipolare Dixon Aufnahmen durch Akquisition der gleich- und gegenphasigen Echos der Wasser- und der Fettprotonen erstellt. In Schritt S3 erfolgt das Anwenden des trainierten CNN auf die in Schritt S2 erstellten Aufnahmedaten zum Minimieren oder vollständigen Entfernen der CSA und Berechnen von korrigierten Aufnahmedaten.

Fig. 6 ist ein Blockschaltbild eines Systems zur Rekonstruktion von MR-Aufnahmen bei Korrektur der CSA. Der MR-Scanner oder das Magnetresonanz-Bildgebungsgerät MR dient zur Erfassung von bipolaren Dixon MR Aufnahmen und ist an eine Verarbeitungseinheit R, die als Rekonstruktionsrechner ausgebildet sein kann, über eine Datenschnittstelle angeschlossen. Der Rekonstruktionsrechner ist weiterhin über eine Netzwerkverbindung mit einem Speicher verbunden, in dem das trainierte CNN gespeichert ist, insbesondere ein U-Netz, wie oben näher beschrieben.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Der Schutzbereich der vorliegenden Erfindung ist durch die nachstehenden Ansprüche gegeben und wird durch die in der Beschreibung erläuterten oder den Figuren gezeigten Merkmale nicht beschränkt.

Für einen Fachmann ist es insbesondere offensichtlich, dass die Erfindung nicht nur für bestimmte MR-Geräte eines Herstellers angewendet werden kann, sondern auch für andere MR-Geräte anderer Hersteller. Des Weiteren können die Bauteile des Magnetresonanz-Bildgebungsgerätes auf mehrere physikalische Produkte verteilt realisiert werden kann. So kann insbesondere der Speicher und/oder die Verarbeitungseinheit auf ein separates oder zusätzliches elektronisches Modul ausgelagert werden.

**Patentansprüche**

1.　Verfahren zur Korrektur chemischer Verschiebungs-Artefakte, CSA, die im Magnetresonanz-DIXON-Verfahren beim Verwenden von bipolaren Auslesegradienten, fast-DIXON-MR, zur Erfassung der gleich- und gegenphasigen Echos auftreten, wobei gleich- und gegenphasig sich auf eine Phasenlage von Wasser- und Fettspins zueinander beziehen, umfassend folgende Verfahrensschritte:

　　(1) Bereitstellen (S1) eines trainierten Convolutional Neural Networks, CNN, das mit gleich- und gegenphasig mit dem DIXON-MR Verfahren aufgenommenen Aufnahmedaten trainiert wurde, umfassend Aufnahmedaten, die CSA in zueinander entgegengesetzten Richtungen des Auslesegradienten beinhalten, und Aufnahmedaten, die CSA nur in einer Richtung des Auslesegradienten beinhalten, so dass das CNN trainiert ist, um die mit dem fast-DIXON-MR erhaltenen Aufnahmedaten so zu transformieren, dass sie nur noch in gleiche Richtung des Auslesegradienten auftretende CSA aufweisen,
　　(2) Erstellen von Steueranweisungen zum Aufnehmen (S2) von zumindest zwei fast-DIXON-MR-Aufnahmedaten durch Akquisition der gleich- und gegenphasigen Echos der Wasser- und der Fettprotonen;
　　(3) Anwenden (S3) des trainierten CNN auf die im vorangehenden Schritt (2) erstellten Aufnahmedaten zum Minimieren oder vollständigen Entfernen der CSA in zueinander entgegengesetzten Richtungen des Auslesegradienten aus den Aufnahmedaten und Berechnen korrigierter Aufnahmedaten, Zuführung der korrigierten Aufnahmedaten zu einem konventionellen Dixon-Algorithmus zur Erzeugung eines Fett und eines Wasserbildes, wobei Fett- und Wasserbild durch eine räumliche Registrierung ausgerichtet werden und die nur noch in die gleiche Richtung des Auslesegradienten auftretende CSA als globale Verschiebung für alle Voxel nur noch im Fettbild auftritt.

2. Verfahren nach Patentanspruch 1, bei dem Schritt (2) vor Schritt (1) ausgeführt wird.

3. Verfahren nach einem der vorangehenden Patentansprüche, wobei es sich bei den Aufnahmedaten um Aufnahmedaten handelt, bei denen Wasser- und Fettspins in Phase sowie außer Phase sind und die CSA an den Grenzflächen zwischen Wasser und Fett vorhanden sind.

4. Verfahren nach einem der vorangehenden Patentansprüche, wobei es sich bei den Aufnahmedaten in Schritt (1) um Bild-Rohdaten in einem k-Raum, rekonstruierte Bilder im Bildraum oder hybride Bilddaten handelt.

5. Verfahren nach einem der vorangehenden Patentansprüche, wobei das CNN eine U-Net-Architektur oder eine GAN-Architektur aufweist.

6. Verfahren nach einem der vorangehenden Patentansprüche, bei dem das Anwenden des trainierten CNN das Bereitstellen von 4-kanaligen oder 6-kanaligen Bilddaten in eine Eingabeschicht des CNN umfasst.

7. Verfahren nach einem der vorangehenden Patentansprüche, bei dem die Inputdaten des CNN reale und imaginäre Anteile der aus den gleichphasigen und gegenphasigen Echos erzeugten Aufnahmedaten umfassen, die miteinander konkateniert sind.

8. Verfahren nach einem der vorangehenden Patentansprüche, bei dem das Training mit monopolaren Aufnahmedaten ohne Zuordnung von bipolaren Aufnahmedaten erfolgt, wobei aus den monopolaren Aufnahmedaten mittels eines Schätzalgorithmus bipolare gleichphasige und gegenphasige Aufnahmedaten erzeugt werden, wobei monopolar einen Auslesegradienten für gleich- und gegenphasiges Echo in gleicher Richtung bezeichnet, und wobei bipolar Auslesegradienten mit entgegengesetzten Richtungen bezeichnet.

9. Verfahren nach Anspruch 8, bei dem der Schätzalgorithmus Rauschen hinzufügt, um Aufnahmedaten mit geringerem Signal-zu-Rausch-Verhältnis zu simulieren.

10. Verfahren nach einem der Ansprüche 8 oder 9, bei dem Aufnahmedaten aus gegenphasigen Echos erzeugt werden, die in um einen von 180° abweichenden Phasenwinkel verschoben sind, indem die Daten der Fettprotonen mit e^(i*Phasenwinkel) multipliziert werden, wobei der Phasenwinkel die tatsächliche Dephasierung im Bereich von 0 bis 2pi ist.

11. System mit einer Verarbeitungseinheit (R) und einem Magnetresonanz-Bildgebungsgerät (MR) zur Ausführung des Verfahrens gemäß einem der vorangegangenen Ansprüche, wobei die Verarbeitungseinheit über eine Datenschnittstelle an das Magnetresonanz-Bildgebungsgerät angeschlossen ist, mit:

   - einem Speicher (MEM) in dem ein trainiertes Convolutional Neural Network, CNN, gespeichert ist, das mit gleich- und gegenphasigen mit dem DIXON-Verfahren aufgenommenen Aufnahmedaten trainiert wurde, umfassend Aufnahmedaten, die CSA in zueinander entgegengesetzten Richtungen des Auslesegradienten beinhalten, und Aufnahmedaten, die CSA nur in einer Richtung des Auslesegradienten beinhalten, so dass das CNN trainiert ist, um die mit dem fast DIXON-MR erhaltenen Aufnahmedaten so zu transformieren, dass sie nur noch in gleiche Richtung des Auslesegradienten auftretende CSA aufweisen
   - wobei das Magnetresonanz-Bildgebungsgerät (MR) zum Erstellen von bipolaren DIXON-MR Aufnahmedaten durch Akquisition der gleich- und gegenphasigen Echos der Wasser- und der Fettprotonen eingerichtet ist;
   - wobei die Verarbeitungseinheit (R) zum Anwenden des trainierten CNN auf die in Schritt (2) erhaltenen Aufnahmedaten zum Minimieren oder vollständigen Entfernen der CSA in zueinander entgegengesetzten Richtungen des Auslesegradienten aus den Aufnahmedaten und zum Berechnen der korrigierten Aufnahmedaten bestimmt ist, sowie zur Zuführung der korrigierten Aufnahmedaten zu einem konventionellen Dixon-Algorithmus zur Erzeugung eines Fett und eines Wasserbildes,

   wobei Fett- und Wasserbild durch eine räumliche Registrierung ausgerichtet werden und die nur noch in die gleiche Richtung des Auslesegradienten auftretende CSA als globale Verschiebung für alle Voxel nur noch im Fettbild auftritt.

12. System nach Anspruch 11 wobei die Verarbeitungseinheit (R) als Rekonstruktionsrechner ausgebildet ist, mit einer Netzwerkverbindung; und

   - und wobei der Speicher (MEM), in dem das trainerte CNN gespeichert ist, eine Netzwerkverbindung zur Verbindung mit dem Rekonstruktionsrechner (R) aufweist.

13. Computerprogramm mit Programmcode-Mitteln, die bewirken, dass das System gemäß Anspruch 11 oder 12 die Verfahrensschritte nach einem der Ansprüche 1-10 ausführt.

**Claims**

1. Method for correcting chemical shift artifacts, CSA, which arise in the magnetic resonance DIXON method when using bipolar readout gradients, fast DIXON MR, to capture the in-phase and opposed-phase echoes, wherein in-phase and opposed-phase relate to a phase relationship between water spins and fat spins, comprising the following method steps:

(1) providing (S1) a trained convolutional neural network, CNN, which has been trained using acquisition data acquired in phase and in phase opposition by the DIXON MR method, comprising acquisition data that contains CSA in mutually opposite directions of the readout gradient, and acquisition data that contains CSA only in one direction of the readout gradient, so that the CNN is trained to transform the acquisition data obtained by the fast DIXON MR such that it exhibits CSA that now arise only in the same direction of the readout gradient;
(2) producing control instructions for acquiring (S2) at least two instances of fast DIXON MR acquisition data, by acquiring the in-phase and opposed-phase echoes of the water protons and fat protons;
(3) applying (S3) the trained CNN to the acquisition data produced in the preceding step (2) in order to minimise the CSA or remove it entirely from the acquisition data in mutually opposite directions of the readout gradient and calculate corrected acquisition data, feed the corrected acquisition data to a conventional Dixon algorithm to generate a fat image and a water image, wherein fat and water images are aligned by spatial registration and the CSA only arising in the same direction of the readout gradient only arises as a global shift for all voxels in the fat image.

2. Method according to claim 1, in which step (2) is performed before step (1).

3. Method according to one of the preceding claims, wherein the acquisition data is the acquisition data in which water spins and fat spins are in phase and out of phase, and the CSA are present at the boundary surfaces between water and fat.

4. Method according to one of the preceding claims, wherein the acquisition data in step (1) is raw image data in k-space, reconstructed images in image space, or hybrid image data.

5. Method according to one of the preceding claims, wherein the CNN has a U-net architecture or a GAN architecture.

6. Method according to one of the preceding claims, in which applying the trained CNN comprises providing 4-channel or 6-channel image data into an input layer of the CNN.

7. Method according to one of the preceding claims, in which the input data of the CNN comprises real and imaginary components of the acquisition data generated from the in-phase and opposed-phase echoes, which are concatenated with each other.

8. Method according to one of the preceding claims, in which the training is performed with monopolar acquisition data without assigning bipolar acquisition data, wherein an estimation algorithm is used to generate bipolar in-phase and opposed-phase acquisition data from the monopolar acquisition data, wherein monopolar relates to one readout gradient for in-phase and opposed-phase echo in the same direction, and wherein bipolar relates to readout gradients with opposite directions.

9. Method according to claim 8, in which the estimation algorithm adds noise in order to simulate acquisition data having a lower signal-to-noise ratio.

10. Method according to one of claims 8 or 9, in which acquisition data is generated from opposed-phase echoes offset by a phase angle other than 180° by multiplying the data for the fat protons by e^(i*phase-angle), where the phase angle is the actual dephasing in the range 0 to 2pi.

11. System with a processing unit (R) and a magnetic resonance imaging device (MR) for performing the method according to one of the preceding claims, wherein the processing unit is connected to the magnetic resonance imaging device via a data interface, having:

- a memory (MEM), in which is stored a trained convolutional neural network, CNN that has been trained with acquisition data acquired in phase and in phase opposition by the DIXON method, comprising acquisition data that contains CSA in mutually opposite directions of the readout gradient, and acquisition data that contains CSA only in one direction of the readout gradient, so that the CNN is trained to transform the acquisition data obtained by the fast DIXON MR such that it exhibits CSA that now arise only in the same direction of the readout gradient;
- wherein the magnetic resonance imaging device (MR) is configured to produce bipolar DIXON MR acquisition data by acquiring the in-phase and opposed-phase echoes of the water protons and fat protons;
- wherein the processing unit (R) is intended to apply the trained CNN to the acquisition data obtained in step (2) in order to minimise the CSA or remove it entirely from the acquisition data in mutually opposite directions of the readout gradient and to calculate the corrected acquisition data, as well as to feed the corrected acquisition data to a conventional Dixon algorithm to generate a fat image and a water image, wherein fat and water images are aligned by spatial registration and the CSA only arising in the same direction of the readout gradient only arises as a global shift for all voxels in the fat image.

12. System according to claim 11, wherein the processing unit (R) is embodied as a reconstruction computer, with a network connection; and

- wherein the memory (MEM), in which the trained CNN is stored, has a network connection for the connection to the reconstruction computer (R).

13. Computer program comprising program code means, which cause the system according to claim 11 or 12 to carry out the method stops according to one of claims 1-10.

**Revendications**

1. Procédé de correction d'artefacts de décalage chimique, CSA, qui se produisent dans le procédé DIXON de résonance magnétique, lors de l'utilisation de gradients de lecture bipolaires, fast-DIXON-RM, pour la détection des échos de même phase et de phase opposée, dans lequel de même phase et de phase opposée se rapportent à une position en phase de spins de l'eau et de spins de matière grasse les uns par rapport aux autres, comprenant les stades de procédé suivants :

(1) se procurer (S1) un réseau neuronal à convolution CNN entraîné, qui a été entraîné par des données d'enregistrement enregistrées de même phase et de phase opposée par le procédé DIXON-RM, comprenant des données d'enregistrement, qui contiennent des CSA dans des sens contraires l'un à l'autre du gradient de lecture et des données d'enregistrement, qui contiennent des CSA seulement dans un sens du gradient de lecture, de sorte que le CNN est entraîné, afin de transformer les données d'enregistrement obtenues par le fast-DIXON-RM, de manière à ce qu'elles n'aient plus que des CSA se produisant dans le même sens du gradient de lecture,
(2) établir des instructions de commande de l'enregistrement (S2) d'au moins deux données d'enregistrement fast-DIXON-RM par acquisition des échos de même phase et de sens opposé des protons de l'eau et des protons de matière grasse ;
(3) appliquer (S3) le CNN entraîné aux données d'enregistrement établies dans le stade (2) précédent, afin de minimiser ou d'éliminer complètement les CSA dans des sens contraires l'un à l'autre du gradient de lecture des données d'enregistrement et
calculer des données d'enregistrement corrigées, apporter les données d'enregistrement corrigées à un algorithme Dixon classique pour la production d'une image de matière grasse et d'eau,

dans lequel on oriente l'image de matière grasse et l'image d'eau par une correspondance dans l'espace et les CSA ne se produisant plus que dans le même sens du gradient de lecture, n'apparaissent plus que dans l'image de matière grasse pour tous les voxels comme décalage global.

2. Procédé suivant la revendication 1, dans lequel on effectue le stade (2) avant le stade (1).

3. Procédé suivant l'une des revendications précédentes, dans lequel les données d'enregistrement sont des données d'enregistrement, dans lesquelles les spins d'eau et les spins de matière grasse sont en phase, ainsi que hors de phase et les CSA sont présents aux surfaces limites entre eau et matière grasse.

4. Procédé suivant l'une des revendications précédentes, dans lequel les données d'enregistrement dans le stade (1) sont des données brutes d'image dans un espace k, des images reconstruites dans l'espace d'image ou des données d'image hybrides.

5. Procédé suivant l'une des revendications précédentes, dans lequel le CNN a une architecture U-Net ou une architecture GAN.

6. Procédé suivant l'une des revendications précédentes, dans lequel l'application du CNN entraîné comprend la mise à disposition de données d'image à 4 canaux ou à 6 canaux dans une couche d'entrée du CNN.

7. Procédé suivant l'une des revendications précédentes, dans lequel les données d'entrée du CNN comprennent des composantes réelles et imaginaires des données d'enregistrement produites à partir des échos de même phase et de phase opposée, qui sont concaténées entre elles.

8. Procédé suivant l'une des revendications précédentes, dans lequel l'apprentissage s'effectue avec des données d'enregistrement monopolaires sans association de données d'enregistrement bipolaires, dans lequel à partir des données d'enregistrement monopolaires, au moyen d'un algorithme d'estimation, on produit des données d'enregistrement bipolaires de même phase et de phase opposée, dans lequel monopolaire désigne un gradient de lecture pour des échos de même sens et de phase opposée dans le même sens, et dans lequel bipolaire désigne des gradients de lecture de sens contraire.

9. Procédé suivant la revendication 8, dans lequel l'algorithme d'estimation ajoute du bruit afin de simuler des données d'enregistrement ayant un rapport signal à bruit plus petit.

10. Procédé suivant l'une des revendications 8 ou 9, dans lequel on produit des données d'enregistrement à partir d'échos de même phase, qui sont décalés d'un angle de phase s'écartant de 180 °, par le fait que les données des protons de matière grasse sont multipliées par e^ (angle de phase i*), dans lequel l'angle de phase et le déphasage réel dans la plage de 0 à 2 pi.

11. Système comprenant une unité (R) de traitement et un appareil (MR) d'imagerie par résonance magnétique pour l'exécution du procédé suivant l'une des revendications précédentes, dans lequel l'unité de traitement est raccordée par une interface de données à l'appareil d'imagerie par résonance magnétique, comprenant :

  - une mémoire (MEM),
  dans laquelle un réseau neuronal à convolution CNN entraîné est mis en mémoire, qui a été entraîné avec des données d'enregistrement de même phase et de phase opposée enregistrées par le procédé DIXON, comprenant des données d'enregistrement, qui contiennent des CSA dans des sens contraires l'un à l'autre du gradient de lecture et des données d'enregistrement, qui contiennent des CSA seulement dans un sens du gradient de lecture, de sorte que le CNN est entraîné, afin de transformer les données d'enregistrement obtenues par le fast DIXON-RM, de manière à ce qu'elles n'aient plus que des CSA se produisant dans le même sens du gradient de lecture ;
  - dans lequel l'appareil (MR) d'imagerie par résonance magnétique est agencé pour l'établissement de données d'enregistrement bipolaires DIXON-RM par acquisition des échos de même phase et de phase opposée des protons d'eau et des protons de matière grasse ;
  - dans lequel l'unité (R) de traitement est destinée à l'application du CNN entraîné aux données d'enregistrement établies dans le stade (2) précédent, afin de minimiser ou d'éliminer complètement les CSA dans des sens contraires l'un à l'autre du gradient de lecture des données d'enregistrement et au calcul des données d'enregistrement corrigées, ainsi qu'apporter les données d'enregistrement corrigées à un algorithme Dixon classique pour la production d'une image de matière grasse et d'eau, dans lequel on oriente l'image de matière grasse et l'image d'eau par une correspondance dans l'espace et les CSA ne se produisant plus que dans le même sens du gradient de lecture, n'apparaissent plus que dans l'image de matière grasse pour tous les voxels comme décalage global.

12. Système suivant la revendication 11, dans lequel l'unité (R) de traitement est constituée en ordinateur de reconstruction, ayant une liaison au réseau ; et

  - dans lequel la mémoire (MEM), dans laquelle le CNN entraîné est mis en mémoire a une connexion au réseau pour la connexion à l'ordinateur (R) de reconstruction.

13. Programme d'ordinateur comprenant des moyens de code de programme, qui font que le système suivant la revendication 11 ou 12 exécute les stades du procédé suivant l'une des revendications 1 à 10.

FIG 1

EP 4 113 150 B1

# FIG 2

EP 4 113 150 B1

FIG 3

```
┌──────────┐      ┌──────────┐
│ 4 x 256  │ ═▷   │  Down    │
│  x 256   │      │  conv1   │
└──────────┘      └──────────┘
                       │
                  ┌──────────┐
                  │  max     │
                  │  pool    │
                  └──────────┘
                       │
                  ┌──────────┐                                            ┌──────────┐      ┌──────────┐
                  │  Down    │──────────────────────────────────────────▶│  concat  │─────▶│   Up     │
                  │  conv2   │                                            │          │      │  conv2   │
                  └──────────┘                                            └──────────┘      └──────────┘
                       │                                                       ▲                 ▲
                  ┌──────────┐                                            ┌──────────┐           │
                  │  max     │                                            │   up     │           │
                  │  pool    │                                            │  sample  │           │
                  └──────────┘                                            └──────────┘           │
                       │                                                       ▲
                  ┌──────────┐      ┌──────────┐      ┌──────────┐      ┌──────────┐
                  │  Down    │─────▶│  concat  │─────▶│   Up     │─────▶│   up     │
                  │  conv3   │      │          │      │  conv1   │      │  sample  │
                  └──────────┘      └──────────┘      └──────────┘      └──────────┘
                       │                 ▲
                  ┌──────────┐      ┌──────────┐      ┌──────────┐      ┌──────────┐
                  │  max     │─────▶│  Down    │─────▶│   up     │──────┘
                  │  pool    │      │  conv4   │      │  sample  │
                  └──────────┘      └──────────┘      └──────────┘
```

┌──────────┐      ┌──────────┐      ┌──────────┐      ┌──────────┐
│  concat  │─────▶│   Up     │ ═▷   │ 4 x 256  │ ⇨    │  Dixon   │
│          │      │  conv3   │      │  x 256   │      │algorithm │
└──────────┘      └──────────┘      └──────────┘      └──────────┘

FIG 4

FIG 5

```
┌──────────┐
│  START   │
└────┬─────┘
     │
     ▼
┌──────────┐
│    S1    │
└────┬─────┘
     │
     ▼
┌──────────┐
│    S2    │
└────┬─────┘
     │
     ▼
┌──────────┐
│    S3    │
└────┬─────┘
     │
     ▼
┌──────────┐
│  ENDE    │
└──────────┘
```

FIG 6

MR

R

U-Netzwerk

MEM

EP 4 113 150 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008057294 A

- EP 3796023 A1

**In der Beschreibung aufgeführte Nicht-Patentliteratur**

- **JINGFEI MA**. Dixon Techniques for Water and Fat Imaging. *Journal of Magnetic Resonance Imaging*, 2008, vol. 28, 543-558
- **VON JINGFEI MA et al.** Fast Spin-Echo Triple-Echo Dixon (fTED) Technique for Efficient T2-Weighted Water and Fat Imaging. *Magnetic Resonance in Medicine*, 2007, vol. 58, 103-109
- **GOLDFARB JW**. Magnitude and Complex Single- and Multi-echo Water Fat Separation via End-to-End Deep Learning. *Proc. Int. Soc. Magn. Res*

- **SON JB et al.** Deep Neural Network for Single-Point Dixon Imaging with Flexible Echo Time. *Proc. Int. Soc. Magn. Res. in Med., ISMRM,11.-16.05.2019, Montreal, Canada*
- **QUELLE: LEINHARD et al.** Quelle. *ISMRM*, 2008
- **RONNEBERGER, OLAF ; FISCHER, PHILIPP ; BROX, THOMAS**. U-Net: Convolutional Networks for Biomedical Image Segmentation. *arXiv:1505.04597*, 2015